# EUROPEAN PATENT APPLICATION

(11) **EP 3 357 909 A1**
(43) Date of publication of application: **08.08.2018**
(21) Application number: 17154412.5
(22) Date of filing: 02.02.2017
(51) Int. Cl.: C07D 211/24, A61K 31/451, A61P 25/28

(54) **CRYSTALLINE 4-[3-(METHYLSULFONYL)PHENYL]-1-PROPYL-PIPERIDINE**

(71) Applicant: Sandoz AG, 4056 Basel (CH)
(72) Inventor: STEFINOVIC, Marijan, 6250 Kundl (AT); MÜHLEGGER, Isabella, 6020 Innsbruck (AT); GRIESSER, Ulrich, 6020 Innsbruck (AT)
(74) Representative: Prüfer & Partner mbB Patentanwälte · Rechtsanwälte

(57) **Abstract**

The present invention relates to crystalline pridopidine free base and a process for its preparation. The invention also relates to a pharmaceutical composition comprising crystalline pridopidine free base and one or more pharmaceutically acceptable ingredients and to the use of said pharmaceutical composition as a medicament, in particular for the treatment of Huntington's disease.

## Description

### FIELD OF THE INVENTION

The present invention relates to crystalline pridopidine free base and a process for its preparation. The invention also relates to a pharmaceutical composition comprising crystalline pridopidine free base and one or more pharmaceutically acceptable ingredients and to said pharmaceutical composition for use as a medicament, in particular for the treatment of motor symptoms associated with Huntington's disease.

### BACKGROUND OF THE INVENTION

Huntington's disease is an autosomal dominant, progressive, neurodegenerative disease that produces a range of cognitive, behavioral and motor deficits. The dopaminergic stabilizer pridopidine is currently in phase III clinical development for the treatment of motor symptoms associated with Huntington's disease. Chemically designated as 4-[3-(methylsulfonyl)phenyl]-1-propyl-piperidine, pridopidine may be represented by the chemical structure as depicted in Formula I:

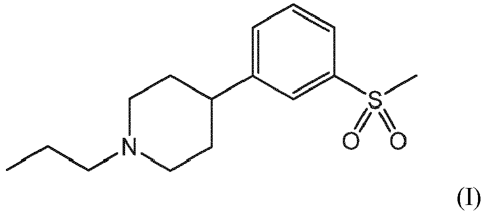

The compound pridopidine is disclosed in WO 01/46145 A1. In Example 6 pridopidine was synthesized as a hydrochloride salt having a melting point of 200 °C by using a method analogous to the one described in Example 1 of the same application.

WO 2006/040155 A1 concerns an alternative method for the synthesis of pridopidine hydrochloride.

Both aforementioned routes of synthesis produce a polymorph of pridopidine hydrochloride designated as "Form I" according to the later application WO 2013/034622 A1 (see page 2, lines 8-12), which itself is directed to a polymorphic form of pridopidine hydrochloride designated as "Form II". The physical properties of pridopidine hydrochloride Form I and Form II and their thermodynamic relationship are investigated in more detail in Zimmermann et al. Cryst. Growth Des. 2012, 12, 2961-2968.

In WO 2011/107583 A1 pridopidine and salts thereof are identified as preferred compounds for the treatment of Hungtington's disease. A long list of possible salts is disclosed on page 8 of the application.

A crystalline form of pridopidine hydrobromide and its preparation are described in WO 2013/086425 A1.

WO 2016/106142 A1 discloses several salts of pridopidine with organic and inorganic acids, and among these salts the pridopidine L-tartrate is described to be ideal for pharmaceutical development (see page 19, lines 7-10).

To date, many pridopidine acid addition salts have been reported in the literature. However, these pridopidine salts suffer from various drawbacks, for example some of these salts exhibit polymorphism, some of them are physically unstable, some are hygroscopic, some even liquefy upon moisture contact, some contain significant amounts of residual organic solvents, some suffer from low crystallinity, some contain amorphous content and/or are associated with safety concerns.

According to WO 2016/106142 A1 page 19, lines 7-10, the L-tartrate salt is ideal for pharmaceutical development. However, the present inventors have found that the L-tartrate salt of WO 2016/106142 A1 is prone to amorphization upon mechanical stress, and is thus problematic for the use in standard pharmaceutical processes such as grinding, milling, sieving, tableting etc.

Hence, there remains a need for the provision of an improved solid form of pridopidine, which enables an easy and economical formulation of a safe and efficacious drug product, and/or which allows for the usage of standard pharmaceutical processes and equipment.

### SUMMARY OF THE INVENTION

The inventors of the present invention found that one or more of the aforementioned drawbacks associated with acid addition salts of pridopidine can be solved by providing pridopidine in form of its crystalline free base. Hence, the invention relates to pridopidine free base in crystalline form, in particular to a crystalline form of pridopidine free base characterized by having a powder X-ray diffractogram comprising reflections at 2-theta angles of (4.4 ± 0.2)°, (13.8 ± 0.2)° and (21.1 ± 0.2)°, when measured at RT with Cu-Kalpha_{1,2} radiation having a wavelength of 0.15419 nm. The invention further relates to a process for the preparation of crystalline pridopidine free base and to a pharmaceutical composition comprising crystalline pridopidine free base, preferably in an effective and/or predetermined amount and one or more pharmaceutically acceptable excipient(s). Moreover, the invention relates to said pharmaceutical composition for use as a medicament, in particular for the treatment of Huntington's disease and in particular the motor symptoms associated with it. Finally, the invention also relates to the use of crystalline pridopidine free base for the preparation of a pharmaceutically acceptable acid addition salt of pridopidine.

### Abbreviations

- PXRD: powder X-ray diffractogram
- SXRD: single X-ray diffraction
- FTIR: Fourier transform infrared
- TGA: thermogravimetric analysis
- GMS: gravimetric moisture sorption
- RT: room temperature
- RH: relative humidity
- w-%: weight percent

### Definitions

The term "pridopidine free base" as used herein refers to the compound with the chemical name 4-[3-(methylsulfonyl)phenyl]-1-propyl-piperidine, which is represented by the chemical structure as depicted in formula II of the present invention. In the present invention pridopidine free base is a form of pridopidine, where the nitrogen of pridopidine is not protonated.

As used herein the term "room temperature" refers to a temperature in the range of from 20 to 30 °C.

As used herein, the term "measured at a temperature in the range of from 20 to 30 °C" refers to a measurement under standard conditions. Typically, standard conditions mean a temperature in the range of from 20 to 30 °C, i.e. at room temperature. Standard conditions can mean a temperature of about 22 °C. Typically, standard conditions can additionally mean a measurement under 20-75% relative humidity, preferably 30-70% relative humidity, more preferably 40-60% relative humidity and most preferably 50% relative humidity.

As used herein "amorphous" refers to a solid form of a compound that is not crystalline. An amorphous compound possesses no long-range order and does not display a definitive X-ray diffraction pattern with reflections.

The term "reflection" with regards to powder X-ray diffraction as used herein, means peaks in an X-ray diffractogram, which are caused at certain diffraction angles (Bragg angles) by constructive interference from X-rays scattered by parallel planes of atoms in solid material, which are distributed in an ordered and repetitive pattern in a long-range positional order. Such a solid material is classified as crystalline material, whereas amorphous material is defined as solid material, which lacks long-range order and only displays short-range order, thus resulting in broad scattering. According to literature, long-range order e.g. extends over at least 103 atoms, whereas short-range order is over a few atoms only (see "Fundamentals of Powder Diffraction and Structural Characterization of Materials" by Vitalij K. Pecharsky and Peter Y. Zavalij, Kluwer Academic Publishers, 2003, page 3).

The term "essentially the same" with reference to PXRD means that variabilities in reflection positions and relative intensities of the reflections are to be taken into account. For example, a typical precision of the 2-Theta values is in the range of ± 0.2° 2-Theta, preferably in the range of ± 0.1° 2-Theta. Thus, a reflection that usually appears at 4.4° 2-Theta for example can appear between 4.2° and 4.6° 2-Theta, preferably between 4.3° and 4.5° 2-Theta on most X-ray diffractometers under standard conditions. Furthermore, one skilled in the art will appreciate that relative intensities of the reflections will show inter-apparatus variability as well as variability due to degree of crystallinity, preferred orientation, sample preparation and other factors known to those skilled in the art and should be taken as qualitative measure only.

Crystalline pridopidine free base may be referred to herein as being characterized by graphical data "as shown in" a figure. Such data include, for example, powder X-ray diffractograms and FTIR spectra. The person skilled in the art understands that factors such as variations in instrument type, response and variations in sample directionality, sample concentration and sample purity may lead to small variations for such data when presented in graphical form, for example variations relating to the exact peak positions and intensities.

The term "solid form" or "physical form" as used herein refers to any crystalline or the amorphous phase of a substance.

The term "anhydrous" or "anhydrate", which may be used interchangeably herein, refers to a solid form, where no water is coordinated in or accommodated by the crystal structure. However, an anhydrate may still comprise residual water due to surface adsorption, solvent inclusions and/ or absorption in disordered regions.

As used herein, the term "non-solvated" refers to a solid form, where no organic solvent is coordinated in or accommodated by the crystal structure. However, a non-solvated solid form may still comprise residual organic solvent(s) due to surface adsorption, solvent inclusions and/ or absorption in disordered regions.

The term "lath-shaped crystal(s)" or "lath(s)" as used herein, when talking about particle shape refers to elongated, thin and blade-like crystals (see "Polymorphism in the Pharmaceutical Industry" edited by Rolf Hilfiker, Wiley-VCH, 2006; Chapter 7, Light Microscopy, Gary Nichols).

The pridopidine can be micronized. The term "micronized" as used herein denotes that a certain matter (in the present invention pridopidine free base) has been subjected to a size reduction process. The review-article "Overview of milling techniques for improving the solubility of poorly water-soluble drugs" (Asian Journal of Pharmaceutical Sciences, Vol. 10, Issue 4, July 2015, pages 255-274) gives an overview on milling techniques and also describes that apart from the particle size, micronization (in particular milling) also alters the surface roughness and shape of particles. For instance, milled particles are rarely spherical or isometric in shape. Milled particles can possess platelet-like shape or needle-shape. The shape of the particles can be determined by any suitable means that is known to a skilled person, e.g. by image analysis techniques, laser diffraction, scanning electron microscopy, transmission electron microscopy and atomic force microscopy. Thus, by analyzing the shape of the size-reduced particles, conclusions can be drawn whether said particle was subjected to a micronization step (size reduction step).

For the purpose of this invention, particle size distribution is determined as the percent volume at each particle size and measured by a laser diffraction method in the context of a circulating aqueous suspension. A Malvern Mastersizer 3000 laser diffraction analyzer equipped with a Hydro EV measurement cell is to be used. Blue laser is on, results are by volume distribution. A measurement sequence consists of eight individual measurements for which the mean value is represented as a histogram.

D80 as used herein means that 80% of the particles (based on volume) are smaller than or equal to the indicated size. D50 as used herein means that 50% of the particles (based on volume) are smaller than or equal to the indicated size. D20 as used herein means that 20% of the particles (based on volume) are smaller than or equal to the indicated size.

A "predetermined amount" as used herein with regard to pridopidine free base refers to the initial amount of pridopidine free base used for the preparation of a pharmaceutical composition having a desired dosage strength of pridopidine.

The term "effective amount" as used herein with regard to pridopidine free base encompasses an amount of pridopidine free base, which causes the desired therapeutic and/or prophylactic effect.

As used herein, the term "about" means within a statistically meaningful range of a value. Such a range can be within an order of magnitude, typically within 10%, more typically within 5%, even more typically within 1% and most typically within 0.1 % of the indicated value or range. Sometimes, such a range can lie within the experimental error, typical of standard methods used for the measurement and/or determination of a given value or range.

As used herein, the term "mother liquor" refers to the solution remaining after crystallization of a solid.

### BRIEF DESCRIPTION OF THE DRAWINGS

**Figure 1****:** illustrates a representative PXRD of the crystalline form of pridopidine free base according to the present invention. The x-axis shows the scattering angle in °2-theta, the y-axis shows the intensity of the scattered X-ray beam in counts of detected photons.
**Figure 2****:** illustrates a representative FTIR spectrum of crystalline pridopidine free base according to the present invention. The x-axis shows the wavenumbers in cm⁻¹, the y-axis shows the relative intensity in percent transmittance.
**Figure 3****:** illustrates a representative TGA curve of crystalline pridopidine free base according to the present invention. The x-axis shows the temperature in degree Celsius (°C), the y-axis shows the mass loss in weight-percent (w-%) referred to the initial mass.
**Figure 4****:** illustrates an overlay of PXRDs of the crystalline form of pridopidine free base of the present invention and of pridopidine L-tartrate of WO 2016/106142 A1 before and after milling. The x-axis shows the scattering angle in °2-theta, the y-axis shows the intensity of the scattered X-ray beam in counts of detected photons. The diffractograms from top to bottom correspond to initial crystalline pridopidine free base, milled pridopidine free base, initial crystalline pridopidine L-tartrate and milled pridopidine L-tartrate salt.
**Figure 5a****:** illustrates a microscopic image of typical pridopidine free base crystals according to the present invention (scale bar 100 micrometer)
**Figure 5b****:** illustrates a microscopic image of typical pridopidine L-tartrate crystals (scale bar 100 micrometer)

### DETAILED DESCRIPTION OF THE INVENTION

The present invention is directed to pridopidine free base in crystalline form.

Pridopidine free base of the present invention is characterized by high chemical purity, high crystallinity and mainly consists of regular lath-shaped crystals with homogenous particle size resulting in excellent powder properties such as flowability, bulk density, compressability etc. In addition, crystalline pridopidine free base of the present invention is physically stable upon mechanical stress.

These favorable properties enable the robust and economical manufacturing of a safe and efficacious drug product comprising crystalline pridopidine free base, since such a drug product can be formulated by using standard pharmaceutical processes such as compression, milling, grinding, sieving, tableting etc. Standard pharmaceutical equipment can be used without running the risk of phase transformations, e.g. polymorph transitions, and/or amorphization of pridopidine during the preparation of a pharmaceutical composition comprising pridopidine.

Different aspects of the invention are described below in further detail by embodiments, without being limited thereto. Each aspect of the invention may be described by one embodiment or by combining two or more of the embodiments.

In a first aspect, the invention relates to crystalline pridopidine free base.

In one embodiment the invention relates to crystalline pridopidine free base, characterized by the chemical structure according to Formula II

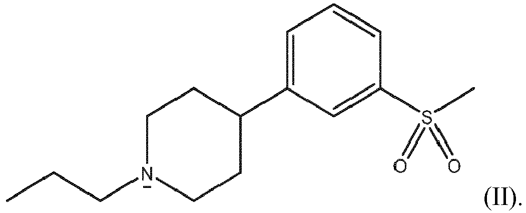

Crystalline pridopidine free base of the present invention may be characterized by analytical methods well known in the field of the pharmaceutical industry for characterizing solids. Such methods comprise but are not limited to PXRD, SXRD, FTIR and TGA. It may be characterized by one of the aforementioned analytical methods or by combining two or more of them. In particular, crystalline pridopidine free base of the invention may be characterized by any one of the following embodiments or by combining two or more of the following embodiments.

Hence, in one embodiment the present invention relates to crystalline pridopidine free base characterized by having a PXRD comprising reflections at 2-Theta angles of:
(4.4 ± 0.2)°, (13.8 ± 0.2)° and (21.1 ± 0.2)°; or
(4.4 ± 0.2)°, (13.8 ± 0.2)°, (15.0 ± 0.2)° and (21.1 ± 0.2)°; or
(4.4 ± 0.2)°, (13.8 ± 0.2)°, (15.0 ± 0.2)°, (16.4 ± 0.2)° and (21.1 ± 0.2)°; or
(4.4 ± 0.2)°, (13.8 ± 0.2)°, (15.0 ± 0.2)°, (16.4 ± 0.2)°, (17.7 ± 0.2)° and (21.1 ± 0.2)°; or
(4.4 ± 0.2)°, (13.8 ± 0.2)°, (15.0 ± 0.2)°, (16.4 ± 0.2)°, (17.7 ± 0.2)°, (18.7 ± 0.2)° and (21.1 ± 0.2)°; or
(4.4 ± 0.2)°, (13.8 ± 0.2)°, (15.0 ± 0.2)°, (16.4 ± 0.2)°, (17.7 ± 0.2)°, (18.7 ± 0.2)°, (20.6 ± 0.2)° and (21.1 ± 0.2)°;
when measured at RT with Cu-Kalpha_{1,2} radiation having a wavelength of 0.15419 nm.

In a preferred embodiment, the present invention relates to crystalline pridopidine free base characterized by having a PXRD comprising reflections at 2-Theta angles of:
(4.4 ± 0.1)°, (13.8 ± 0.1)° and (21.1 ± 0.1)°; or
(4.4 ± 0.1)°, (13.8 ± 0.1)°, (15.0 ± 0.1)° and (21.1 ± 0.1)°; or
(4.4 ± 0.1)°, (13.8 ± 0.1)°, (15.0 ± 0.1)°, (16.4 ± 0.1)° and (21.1 ± 0.1)°; or
(4.4 ± 0.1)°, (13.8 ± 0.1)°, (15.0 ± 0.1)°, (16.4 ± 0.1)°, (17.7 ± 0.1)° and (21.1 ± 0.1)°; or
(4.4 ± 0.1)°, (13.8 ± 0.1)°, (15.0 ± 0.1)°, (16.4 ± 0.1)°, (17.7 ± 0.1)°, (18.7 ± 0.1)° and (21.1 ± 0.1)°; or
(4.4 ± 0.1)°, (13.8 ± 0.1)°, (15.0 ± 0.1)°, (16.4 ± 0.1)°, (17.7 ± 0.1)°, (18.7 ± 0.1)°, (20.6 ± 0.1)° and (21.1 ± 0.1)°;
when measured at RT with Cu-Kalpha_{1,2} radiation having a wavelength of 0.15419 nm.

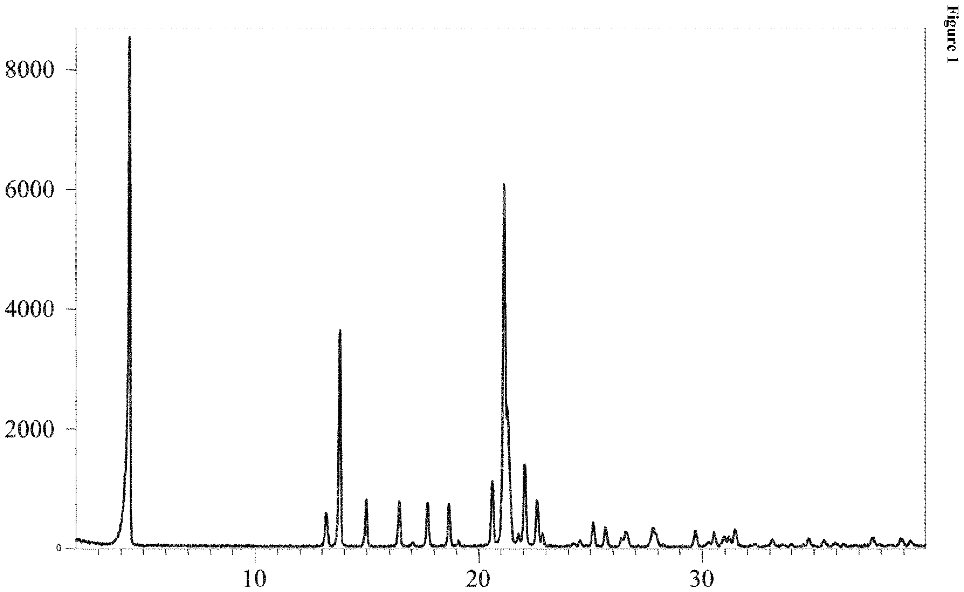

In another embodiment, the present invention relates to crystalline pridopidine free base characterized by having a PXRD essentially the same as shown in Figure 1 of the present invention, when measured at RT with Cu-Kalpha_{1,2} radiation having a wavelength of 0.15419 nm.

In a further embodiment, the present invention relates to crystalline pridopidine free base characterized by having an FTIR spectrum comprising peaks at wavenumbers of:
(2943 ± 2) cm⁻¹, (1599 ± 2) cm⁻¹, (1471 ± 2) cm⁻¹, (1294 ± 2) cm⁻¹ and (1138 ± 2) cm⁻¹; or
(2943 ± 2) cm⁻¹, (1599 ± 2) cm⁻¹, (1471 ± 2) cm⁻¹, (1294 ± 2) cm⁻¹, (1138 ± 2) cm⁻¹ and (1092 ± 2) cm⁻¹; or
(2943 ± 2) cm⁻¹, (1599 ± 2) cm⁻¹, (1471 ± 2) cm⁻¹, (1294 ± 2) cm⁻¹, (1138 ± 2) cm⁻¹, (1092 ± 2) cm⁻¹ and (970 ± 2) cm⁻¹; or
(2943 ± 2) cm⁻¹, (1599 ± 2) cm⁻¹, (1471 ± 2) cm⁻¹, (1294 ± 2) cm⁻¹, (1138 ± 2) cm⁻¹, (1092 ± 2) cm⁻¹, (970 ± 2) cm⁻¹ and (757 ± 2) cm⁻¹; or
(2943 ± 2) cm⁻¹, (1599 ± 2) cm⁻¹, (1471 ± 2) cm⁻¹, (1294 ± 2) cm⁻¹, (1138 ± 2) cm⁻¹, (1092 ± 2) cm⁻¹, (970 ± 2) cm⁻¹, (757 ± 2) cm⁻¹ and (684 ± 2); or
(2943 ± 2) cm⁻¹, (1599 ± 2) cm⁻¹, (1471 ± 2) cm⁻¹, (1294 ± 2) cm⁻¹, (1203 ± 2) cm⁻¹, (1138 ± 2) cm⁻¹, (1092 ± 2) cm⁻¹, (970 ± 2) cm⁻¹, (757 ± 2) cm⁻¹ and (684 ± 2);
when measured at RT with a diamond attenuated total reflection cell.

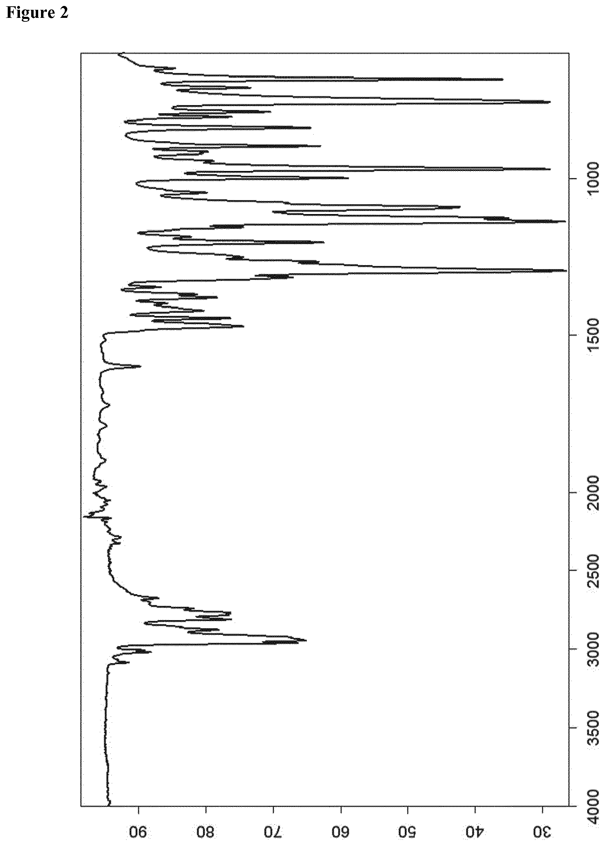

In another embodiment, the present invention relates to crystalline pridopidine free base characterized by having an FTIR spectrum essentially the same as shown in Figure 2 of the present invention, when measured at RT with a diamond attenuated total reflection cell.

In still a further embodiment, the present invention relates to crystalline pridopidine free base characterized by having the triclinic space group symmetry *P*1̅ and the following unit cell parameters:
a = 5.3664 Angstrom;
b = 6.9151 Angstrom;
c = 19.8133 Angstrom;
alpha = 94.946°;
beta = 90.540°;
gamma = 91.393°;
when measured at a temperature of 173 K with Mo-Kalpha_{1,2} radiation having a wavelength of 0.71073 Angstrom.

In another embodiment, the present invention relates to crystalline pridopidine free base characterized by having a TGA curve showing a mass loss of not more than 0.5 w-%, preferably of not more than 0.1 w-%, more preferably of not more than 0.05 w-% up to a temperature of 100 °C, based on the weight of the crystalline form, when measured at a heating rate of 5 K/min.

In a particular preferred embodiment, the present invention relates to crystalline pridopidine free base as defined hereinabove, which is non-solvated.

In another preferred embodiment, the present invention relates to crystalline pridopidine free base as defined hereinabove, which is anhydrous.

In a further embodiment the crystalline pridopidine free base of the present invention may be characterized by comprising crystals having lath shape.

In another aspect the invention relates to a composition comprising at least 90 w-%, preferably at least 95 w-% of the crystalline pridopidine free base as defined hereinabove, based on the total weight of the composition.

In a preferred embodiment, the invention relates to a composition comprising at least 90 w-%, preferably at least 95 w-% of the crystalline pridopidine free base as defined hereinabove and less than 5 w-% amorphous pridopidine, such as less than 2 w-% amorphous pridopidine.

To date, many pridopidine acid addition salts have been reported in the literature. WO 2016/106142 A1 discusses in some detail the drawbacks of the reported pridopidine salts e.g. it is shown in Example 3 that they are hygroscopic (e.g. the hydrochloride, gentisate, glycolate, L-malate, succinate and hemisuccinate) or even liquefy upon moisture contact (e.g. the hydrochloride, glyolate, succinate and hemisuccinate), exhibit polymorphism (e.g. the hydrochloride, besylate and fumarate) and/or raise safety concerns (e.g. the hydrobromide,). On page 19, lines 7-10, it is finally concluded, that based on its physical properties, the L-tartrate salt is ideal for development.

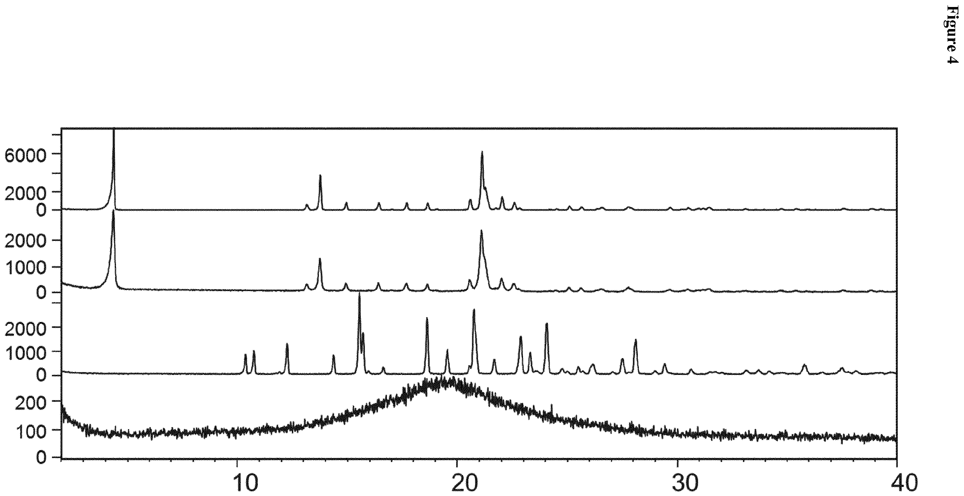

However, it was found by the present inventors that the L-tartrate salt of WO 2016/106142 A1 is prone to amorphization upon mechanical stress e.g. after milling. As can be seen from Example 6 and Figure 4 hereinafter, milling of crystalline pridopidine L-tartrate led to amorphization of said salt resulting in a PXRD showing only an amorphous halo.

In contrast, it can be seen from Example 6 and Figure 4 hereinafter that the PXRD of crystalline pridopidine free base of the present invention remained unchanged after having applied the material to the same milling conditions. The fact that crystalline pridopidine free base of the present invention preserves its high crystallinity even upon significant mechanical stress allows for the use of standard pharmaceutical processes and standard equipment without risk of phase transformations, e.g. polymorph transitions and/or amorphization, which renders the formulation process robust and economical. The use of pridopidine free base also enables the preparation of compressed pharmaceutical dosage forms, such as tablets, comprising crystalline pridopidine, wherein no amorphous pridopidine is present in said compressed dosage form, e.g. the tablet.

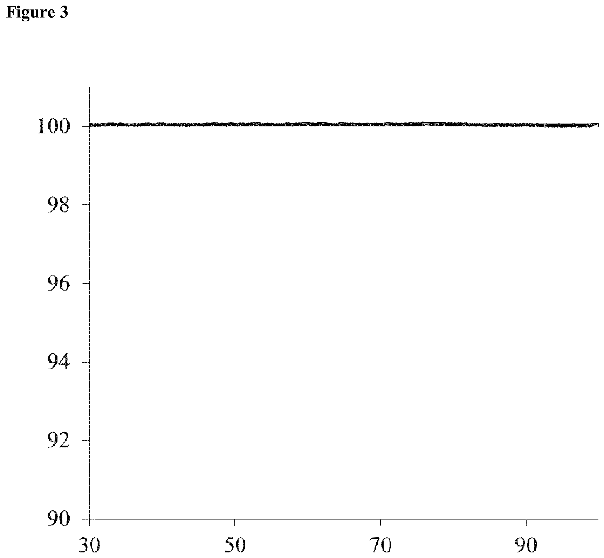

Furthermore, according to TGA analysis presented in WO 2016/106142 A1 the crystalline L-tartrate salt loses 0.5 w-%, when heated from 25 to 150 °C at a rate of 10 K/min, based on the weight of the crystalline pridopidine L-tartrate (see page 16, lines 15-16 and Figure 3 of WO 2016/106142 A1). According to the DVS curve presented in Figure 4 of WO 2016/106142 A1 the anhydrous L-tartrate salt contains practically no water at relative humidities below about 40%. Considering the dry atmosphere of the furnace, which is purged with Helium (see page 11, lines 12-14) and the fact that the L-tartrate salt contains no water at such dry conditions, the mass loss of about 0.5 w-% can be purely assigned to residual organic solvent(s). This indicates, that the removal of residual organic solvents is not straightforward.

While a residual solvent content of 0.5 w-% may be still acceptable with regard to regulatory requirements, organic solvents should nevertheless be reduced to the extent possible, since no therapeutic effect is associated with said solvents.

Pridopidine free base of the present invention shows no significant weight loss (only 0.05 w-% up to a temperature of 100 °C), when heated from 25 °C to 100 °C in a TGA experiment. This shows that practically no residual organic solvents are present (see Example 4 and Figure 3 hereinafter).

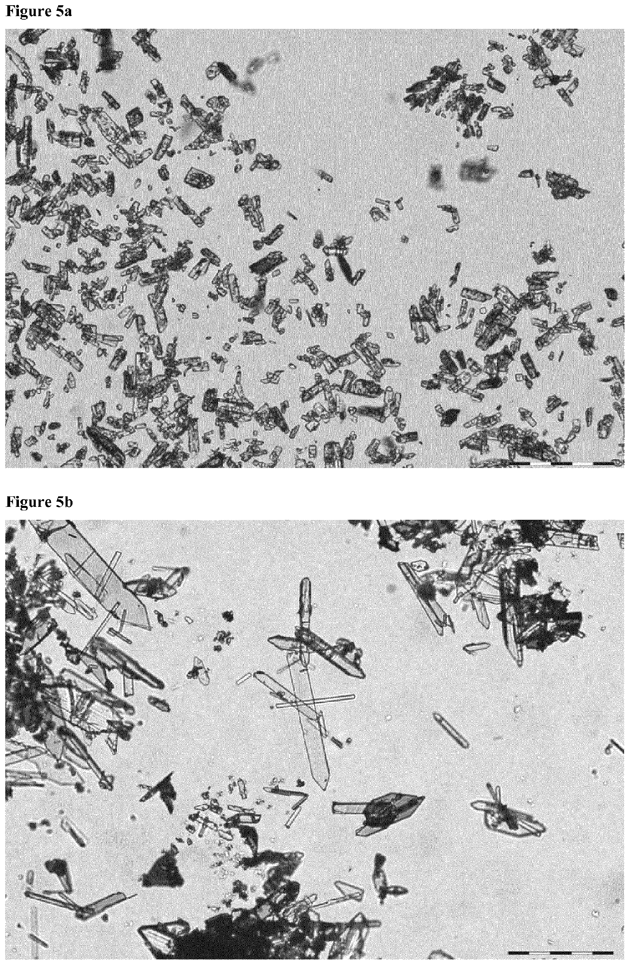

Finally, Figure 5b displays typical crystals of pridopidine L-tartrate. It can be seen that these crystals are inhomogeneous in particle shape and size. In contrast to the irregular shaped and sized pridopidine L-tartrate crystals, crystalline pridopidine free base according to the present invention is characterized by comprising mainly regularly lath-shaped crystals of homogenous particle size (see Figure 5a herein) resulting in improved powder properties such as higher bulk density, better compressibility and better flowability. This clearly favors crystalline pridopidine free base of the present invention to be used for pharmaceutical processing, e.g. for tableting or capsule filling processes.

Also from a process perspective, pridopidine free base is favored since salt formation can mean an additional synthesis step, may cause corrosion and pitting of steel equipment when applying strong acids, and pose a risk for the processors.

Hence, in a further aspect, the invention relates to a process for the preparation of crystalline pridopidine free base as defined above comprising:
(i) reacting one or more acid addition salt(s) of pridopidine with a base in the presence of an aqueous solvent; and
(ii) optionally slurrying the mixture obtained in step (i); and
(iii)optionally separating at least a part of the crystals obtained in step (i) or (ii) from their mother liquor; and
(iv)optionally drying the crystals obtained in any one of previous steps (i) to (iii);

Suitable pridopidine acid addition salts, which may be applied as starting materials in step (i) of the above described process may be selected from the group consisting of acetate, adipate, alginate, ascorbate, aspartate, benzenesulfonate, benzoate, camphorate, camphor-10-sulfonate, caprate, caprylate, carbonate, hydrogencarbonate, cinnamate, citrate, cyclamate, dodecylsulfate, ethane-1,2-disulfonate, esylate, 2-hydroxyesylate, formate, fumarate, galactarate, gentisate, gluconate, glucuronate, glutamate, glutarate, glycolate, hippurate, hydrobromide, hydrochloride, isobutyrate, lactate, lactobionate, laurate, maleate, malate, malonate, mandelate, mesylate, naphthalene-1,5-disulfonate, naphthalene-2-sulfonate, nicotinate, nitrate, oleate, orate, oxalate, palmitate, pamoate, phosphate, propionate, pyroglutamate, salicylate, 4-aminosalicylate, stearate, succinate, sulfate, tartrate, thiocyanate and tosylate. Preferably, the acid addition salt is selected from the group consisting of hydrobromide, hydrochloride and tartrate e.g. L-tartrate and most preferably pridopidine hydrochloride is applied as starting material in step (i) of the above described process. Such salts may be formed by procedures well known and described in the art e.g. by reacting equimolar amounts of pridopidine and the corresponding acid in the presence of a suitable solvent.

Suitable bases, which may be applied in step (i) of the above described process may be selected from the group consisting of ammonia, *L*-arginine, benethamine, benzathine, betaine, calcium hydroxide, choline, dimethylaminoethanol, diethanolamine, diethylamine, 2-diethylaminoethanol, ethanolamine, ethylenediamine, *N*-methylglucamine, hydrabamine, 1*H-*imidazole, lysine, magnesium hydroxide, 4-(2-hydroxyethyl)-morpholine, piperazine, potassium carbonate, potassium hydroxide, 1-(2-hydroxyethyl)-pyrrolidine, sodium bicarbonate, sodium hydroxide, triethanolamine, tromethamine and zinc hydroxide. Preferably, the base is selected from the group consisting ammonia, *L*-arginine, calcium hydroxide, choline, *N*-methylglucamine, lysine, magnesium hydroxide, potassium carbonate, potassium hydroxide, sodium bicarbonate, sodium carbonate and sodium hydroxide. Most preferably sodium hydroxide is used.

The aqueous solvent in step (i) of the above described procedure may comprise water and optionally one or more water-miscible organic solvent(s) selected from the group consisting of acetone, acetonitrile, 2-butanone, diethylene glycol, 1,2-dimethoxyethane, *N,N-*dimethylformamide, dimethylsulfoxide, ethanol, ethylene glycol, 2-methyltetrahydrofurane, nitromethane, 1-propanol, 2-propanol and tetrahydrofuran. Preferably, water is the only solvent present.

In a particular embodiment, the invention relates to a process for the preparation of crystalline pridopidine free base as defined above comprising reacting pridopidine hydrochloride with sodium hydroxide in the presence of water.

The molar ratio of pridopidine acid addition salt and base applied in step (i) of the above described procedure is preferably in the range of from about 1.0 (pridopidine acid addition salt): 0.5 to 5.0 (base), more preferably from about 1.0 (pridopidine acid addition salt): 0.8 to 2.5 (base) and most preferably from about 1.0 (pridopidine acid addition salt): 1.0 to 2.0 (base).

The reaction is preferably carried out at a temperature in the range of from about 5 to 60 °C, more preferably from about 10 to 40 °C and most preferably at about RT.

The final pridopidine concentration of the mixture obtained in step (i) is preferably in the range of from about 25 to 250 g/L, more preferably from about 30 to 150 g/L and most preferably from about 40 to 60 g/L.

The mixture obtained in step (i) is optionally slurried in step (ii), wherein slurrying in the context of the present invention relates to any motion of the mixture comprising pridopidine, which is caused by stirring, shaking and/or ultrasonic irradiation. Slurrying may be performed for a period in the range of from about 0.5 to 48 hours, preferably from about 1 to 24 hours, more preferably from about 2 to 12 hours.

The obtained pridopidine free base crystals or at least a part thereof may optionally be separated from their mother liquor by any conventional method such as filtration or centrifugation, most preferably by filtration. Optionally, the isolated crystals may be washed with a solvent, preferably with the same solvent or solvent mixture as used in step (i).

Finally, the pridopidine free base crystals may optionally be dried at a temperature of about 60 °C or less, preferably of about 50 °C or less, more preferably of about 40 °C or less, for example at about 40 °C or at about room temperature. Drying may be performed for a period in the range of from about 1 to 72 hours, preferably from about 2 to 48 hours, more preferably from about 4 to 24 hours and most preferably from about 6 to 18 hours. Drying may be performed at ambient pressure and/or under vacuum preferably at about 100 mbar or less, more preferably at about 50 mbar or less and most preferably at about 30 mbar or less, for example at about 20 mbar or less.

In another aspect, the invention relates to the use of the crystalline pridopidine free base of the present invention for the preparation of a pharmaceutical composition.

In a further aspect the invention relates to a pharmaceutical composition comprising the crystalline pridopidine free base of the present invention, preferably in an effective and/or predetermined amount or the composition comprising crystalline pridopidine free base as defined above and one or more pharmaceutically acceptable excipient(s).

In a preferred embodiment, the pharmaceutical composition of the present invention is an oral solid dosage form such as a tablet or a capsule and most preferably the pharmaceutical composition is a tablet.

In another aspect, the present invention relates to the pharmaceutical composition as defined above for use as a medicament.

In still another aspect, the invention relates to the pharmaceutical composition as defined above for the treatment of motor symptoms associated with Huntington's disease.

In a further aspect, the invention relates to a method for the treatment of motor symptoms associated with Huntington's disease comprising administering a predetermined and/or effective amount of crystalline pridopidine free base as defined herein or a pharmaceutical composition comprising a predetermined and/or effective amount of crystalline pridopidine free base as described herein to a patient in need of such a treatment.

Crystalline pridopidine free base enables the preparation of compressed pharmaceutical dosage forms, such as tablets, comprising only crystalline pridopidine, but not amorphous pridopidine. Therefore, the present invention also relates to a compressed pharmaceutical dosage form, such as a tablet, wherein the compressed pharmaceutical dosage form contains only crystalline pridopidine, such as crystalline pridopidine free base, but no detectable amounts of amorphous pridopidine.

Crystalline pridopidine free base remains crystalline when a size reduction process is applied. Thus it enables the preparation of pharmaceutical dosage forms comprising micronized pridopidine, which micronized pridopidine is crystalline, and wherein the dosage form does not contain detectable amounts of amorphous pridopidine. Therefore, the present invention also relates to a pharmaceutical dosage form comprising micronized pridopidine, such as micronized pridopidine free base, which micronized pridopidine is crystalline, and wherein the dosage form does not contain detectable amounts of amorphous pridopidine.

The presence of amorphous pridopidine can be detected by the presence of a distinct halo in the powder x-ray diffraction pattern obtained from a tested sample. Alternatively or additionally, the presence of amorphous pridopidine can be detected by the presence of a glass transition, which is indicated by a characteristic shift in the endothermic direction in the DSC curve obtained from a tested sample.

Crystalline prodopidine free base and micronized crystalline pridopidine free base can be produced with a much more uniform particle size distribution compared to pridopidine L-tartrate, while at the same time maintaining a high degree of crystallinity. The present invention therefore also relates to crystalline pridopidine, such as crystalline pridopidine free base, having a particle size distribution wherein the d80 value is at most 5.0 times the d20 value, such as at most 4.0 x d20, for example at most 3.0 x d20, and wherein the pridopidine does not contain detectable amounts of amorphous pridopidine. Preferably the d50 is at most 50 µm, such as at most 40 µm, for example in a range of from 10 µm to 40 µm.

The fact that crystalline pridopidine free base of the present invention is easy to isolate and dry after synthesis and characterized by high chemical purity also renders this form an especially suitable intermediate for the purification of acid addition salts of pridopidine.

Hence, in still another aspect, the invention relates to the use of crystalline pridopidine free base as described herein for the preparation of a pharmaceutically acceptable acid addition salt of pridopidine.

In one embodiment, the pharmaceutically acceptable acid addition salt of pridopidine is selected from the group consisting of pridopidine besylate, fumarate, gentisate, glycolate, hydrobromide, hydrochloride, *L*-malate, naphthalene -2-sulfonate, oxalate, succinate, *L*-tartrate and tosylate.

In a particular preferred embodiment, the pharmaceutically acceptable acid addition salt of pridopidine is selected from the group consisting of pridopidine hydrobromide, hydrochloride, *and L*-tartrate. Most preferably the pharmaceutically acceptable acid addition salt of pridopidine is pridopidine *L*-tartrate.

### EXAMPLES

The following non-limiting examples and analytical methods which have been applied for the generation of analytical data are illustrative for the disclosure:

### Example 1: crystalline pridopidine free base - preparation method

A solution of pridopidine hydrochloride (2.00 g, e.g. prepared according to the procedure disclosed in WO 2006/040155 A1) in deionized water (20 mL) was added dropwise to aqueous sodium hydroxide (0.1 N, 10 mL) at room temperature under stirring. After complete addition, the obtained suspension was further stirred at room temperature for 2 hours. Finally, the pridopidine free base crystals were collected by filtration, washed with water (5-times, 3 mL) and dried under vacuum (30mbar) at 40 °C for 19 hours.
Yield: 1.77 g (95% of theory)

### Example 2: crystalline pridopidine free base - powder X-ray diffractometry

Powder X-ray diffraction was performed with an X'Pert PRO diffractometer (PANalytical, Almelo, The Netherlands) equipped with a theta/theta coupled goniometer in transmission geometry, programmable XYZ stage with well plate holder, Cu-Kalpha_{1,2} radiation source (wavelength 0.15419 nm) with a focusing mirror, a 0.5° divergence slit, a 0.02° soller slit collimator and a 0.5° anti-scattering slit on the incident beam side, a 2 mm anti-scattering slit, a 0.02° soller slit collimator, a Ni-filter and a solid state PIXcel detector on the diffracted beam side. The diffractogram was recorded at a tube voltage of 40 kV, tube current of 40 mA, applying a stepsize of 0.013° 2-Theta with 40 seconds per step in the angular range of 2° to 40° 2-Theta.

A typical precision of the 2-Theta values is in the range of ± 0.2° 2-Theta, preferably in the range of ± 0.1° 2-Theta. Thus, the reflection of the crystalline form of pridopidine free base of the present invention that appears for example at 4.4° 2-Theta can appear between 4.2° and 4.6° 2-Theta, preferably between 4.3° and 4.5° 2-Theta on most X-ray diffractometers under standard conditions.

A representative powder X-ray diffractogram of the crystalline form of pridopidine free base of the present invention is displayed in figure 1 herein and the corresponding reflection list is provided in table 1 below.

**Table 1: Reflection list and corresponding relative intensities of the crystalline form of pridopidine free base of the present invention between 2.0 and 30.0° 2-Theta**

| Angle [± 0.2 °2-Theta] | Relative Intensity [%] | Angle [± 0.2 °2-Theta] | Relative Intensity [%] |
|---|---|---|---|
| 4.4 | 100 | 21.8 | 3 |
| 13.2 | 6 | 22.0 | 16 |
| 13.8 | 42 | 22.6 | 9 |
| 15.0 | 9 | 22.8 | 2 |
| 16.5 | 9 | 24.5 | 1 |
| 17.1 | 1 | 25.1 | 5 |
| 17.7 | 8 | 25.7 | 4 |
| 18.7 | 8 | 26.4 | 2 |
| 19.1 | 1 | 26.6 | 3 |
| 20.6 | 13 | 27.8 | 4 |
| 21.1 | 70 | 29.7 | 3 |
| 21.3 | 27 | | |

### Example 3: crystalline pridopidine free base - FTIR spectroscopy

The FTIR spectrum was recorded (obtained) on an MKII Golden Gate™ Single Reflection Diamond ATR cell with a Bruker Tensor 27 FTIR spectrometer with 4 cm⁻¹ resolution at RT. To record a spectrum a spatula tip of a sample was applied to the surface of the diamond in powder form. Then the sample was pressed onto the diamond with a sapphire anvil and the spectrum was recorded. A spectrum of the clean diamond was used as background spectrum. A typical precision of the wavenumber values is in the range of about ± 2 cm⁻¹. Thus, the infrared peak of crystalline pridopidine free base of the present invention that appears at 1294 cm⁻¹ can appear between 1292 and 1296 cm⁻¹ on most infrared spectrometers under standard conditions.

A representative FTIR spectrum of crystalline pridopidine free base of the present invention is displayed in figure 2 and the corresponding peak list from 4000 to 600 cm⁻¹ is provided in table 2 below.

**Table 2: FTIR peak list of crystalline pridopidine free base between 4000 and 600 cm⁻¹**

| **Wavenumber [± 2 cm⁻¹]** | **Wavenumber [± 2 cm⁻¹]** | **Wavenumber [± 2 cm⁻¹]** | **Wavenumber [± 2 cm⁻¹]** |
|---|---|---|---|
| 3085 | 1471 | 1251 | 944 |
| 3018 | 1446 | 1203 | 915 |
| 2959 | 1421 | 1186 | 897 |
| 2943 | 1398 | 1157 | 838 |
| 2875 | 1380 | 1138 | 805 |
| 2809 | 1369 | 1127 | 788 |
| 2769 | 1346 | 1092 | 757 |
| 2741 | 1315 | 1046 | 711 |
| 2676 | 1294 | 998 | 684 |
| 1599 | 1265 | 970 | 650 |

### Example 4: crystalline pridopidine free base - thermogravimetric analysis

TGA was performed with thermogravimetric-system TGA-7 using Pyris-Software for Windows NT, (Perkin-Elmer, Norwalk, Ct., USA). The sample (2.99 mg) was weighed in a platinum-sample holder (50 µL) and heated from room temperature to 100 °C at a heating rate of 5 K/min. Nitrogen was used as the purge gas (sample purge: 20 mL/min, balance purge: 40 mL/min).

Crystalline pridopidine free base of the present invention showed practically no mass loss (0.05 w-% up to a temperature of 100 °C) indicating the presence of an anhydrous and solvent free form. The corresponding TGA curve is displayed in figure 3 herein.

### Example 5: crystalline pridopidine free base - single crystal X-ray diffraction

Intensity data were collected at 173 K, using Mo radiation (λ = 0.71073 Å), on an Oxford Diffraction Gemini-R Ultra diffractometer operated by the CrysAlisPro software (Rigaku OD, 2015). The data were corrected for absorption effects by means of comparison of equivalent reflections. The structure was solved with the direct methods procedure implemented in SHELXT and refined by full-matrix least squares on F2 using SHELXL-2014 [Sheldrick, Acta Cryst. A71 (2015), 3-8 and C71 (2015), 3-8]. Non-hydrogen atoms were located in difference maps and refined anisotropically. Hydrogen atoms were located in difference maps and refined using riding models.

### Example 6: Physical stability upon mechanical stress

Crystalline pridopidine free base of the present invention and the crystalline form of pridopidine L-tartrate of WO 2016/106142 A1 (prepared according to the procedure disclosed in example 2 of WO 2016/106142 A1) were subjected to milling using a Retsch Mixer mill MM 301 (Retsch GmbH, Germany) respectively. For this purpose, the samples (50 mg) were each placed in 10 mL grinding jars each fitted with 2 grinding balls (diameter: 10 mm) and milled for 10 minutes at a frequency of 20 oscillations per second. The physical stabilities of the samples were determined by measuring PXRDs of the initial and the milled samples. As can be seen from the PXRD overlay presented in figure 4 herein the PXRD of milled pridopidine free base corresponds well to the PXRD of the initial sample. The presence of sharp reflections indicates that pridopidine free base is still of high crystallinity and that no significant amorphous portions are present. In contrast, the PXRD of the milled pridopidine L-tartrate lacks reflections but only consists of an amorphous halo, which indicates that this sample turned completely amorphous after the milling process.

### Example 7: Particle shape and particle size distribution

The particle shapes and particle size distributions of crystalline pridopidine free base of the present invention and crystalline pridopidine L-tartrate of WO 2016/106142 A1 were investigated by polarized light microsopy using an Olympus BH2 polarization microscope (Olympus GmbH, Austria). Microscopic images were taken with an Olympus DP71 digital camera (Olympus GmbH, Austria).

As can be seen from the photomicrographic image displayed in figure 5a hereinafter, crystalline pridopidine free base according to the present invention is characterized by comprising mainly regularly lath-shaped crystals of homogenous particle size.

In contrast, from the photomicrographic image displayed in figure 5a hereinafter, which displays typical crystals of pridopidine L-tartrate it can be seen that these crystals are much more inhomogeneous with regard to morphology and particle size.

## Claims

1. Crystalline 4-[3-(methylsulfonyl)phenyl]-1-propyl-piperidine (pridopidine free base).

2. The crystalline pridopidine free base of claim 1, **characterized by** the chemical structure according to formula II

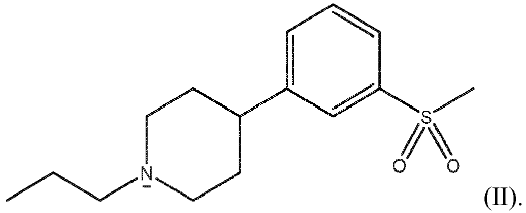

3. The crystalline pridopidine free base of claim 1 or 2 **characterized by** having a powder X-ray diffractogram comprising reflections at 2-Theta angles of (4.4 ± 0.2)°, (13.8 ± 0.2)° and (21.1 ± 0.2)°, when measured at a temperature in the range of from 20 to 30 °C with Cu-Kalpha_{1,2} radiation having a wavelength of 0.15419 nm.

4. The crystalline pridopidine free base of claim 3 **characterized by** having a powder X-ray diffractogram comprising further reflections at 2-Theta angles of (15.0 ± 0.2)° and/or (16.4 ± 0.2)°, when measured at a temperature in the range of from 20 to 30 °C with Cu-Kalpha_{1,2} radiation having a wavelength of 0.15419 nm.

5. The crystalline pridopidine free base as defined in any one of the preceding claims **characterized by** having a Fourier transform infrared spectrum comprising peaks at wavenumbers of (2943 ± 2) cm⁻¹, (1599 ± 2) cm⁻¹, (1471 ± 2) cm⁻¹, (1294 ± 2) cm⁻¹ and (1138 ± 2) cm⁻¹, when measured at a temperature in the range of from 20 to 30 °C with a diamond attenuated total reflection cell.

6. The crystalline pridopidine free base as defined in any one of the preceding claims **characterized by** having the triclinic space group symmetry *P*1̅ and the following unit cell parameters:
a = 5.3664 Angstrom;
b = 6.9151 Angstrom;
c = 19.8133 Angstrom;
alpha = 94.946°;
beta = 90.540°;
gamma = 91.393°;
when measured at a temperature of 173 K with Mo-Kalpha_{1,2} radiation having a wavelength of 0.71073 Angstrom.

7. A composition comprising at least 90 w-%, preferably at least 95 w-% of the crystalline pridopidine free base as defined in anyone of the preceding claims, based on the total weight of the composition.

8. A process for the preparation of the crystalline pridopidine free base as defined in any one of claims 1 to 6 or the composition of claim 7 comprising the steps:
(i) reacting one or more acid addition salt(s) of pridopidine with a base in the presence of an aqueous solvent;
(ii) optionally slurrying the mixture obtained in step (i);
(iii)optionally separating at least a part of the crystals obtained in step (i) or (ii) from their mother liquor; and
(iv)optionally drying the crystals obtained in any one of previous steps (i) to (iii);

9. Use of the crystalline pridopidine free base as defined in any one of claims 1 to 6 or the composition of claim 7 for the preparation of a pharmaceutical composition.

10. A pharmaceutical composition comprising crystalline pridopidine free base as defined in any one of claims 1 to 6 or the composition of claim 7 and one or more pharmaceutically acceptable excipient(s).

11. The pharmaceutical composition as defined in claim 10 for use as a medicament.

12. The pharmaceutical composition as defined in claim 10 for the treatment of motor symptoms associated with Huntington's disease.

13. Use of the crystalline pridopidine free base as defined in any one of claims 1 to 6 or the composition of claim 7 for the preparation of a pharmaceutically acceptable acid addition salt of pridopidine.

14. The use according to claim 13, wherein the pharmaceutically acceptable acid addition salt of pridopidine is selected from the group consisting of pridopidine hydrobromide, pridopidine hydrochloride and pridopidine L-tartrate.
